# EUROPEAN PATENT SPECIFICATION

(11) **EP 4 395 626 B1**
(45) Date of publication and mention of the grant of the patent: **23.07.2025**
(21) Application number: 22761609.1
(22) Date of filing: 09.08.2022
(51) Int. Cl.: A61B 5/00, A61B 5/346

(54) **APPARATUS AND METHOD FOR ELECTROCARDIOGRAM ("ECG") SIGNAL ANALYSIS AND ARTIFACT DETECTION**
VORRICHTUNG UND VERFAHREN ZUR ELEKTROKARDIOGRAMMSIGNALANALYSE UND ARTEFAKTDETEKTION
APPAREIL ET PROCÉDÉ D'ANALYSE DE SIGNAL D'ÉLECTROCARDIOGRAMME ("ECG") ET DE DÉTECTION D'ARTÉFACT

(30) Priority: 30.08.2021 US 202163238604 P; 29.07.2022 US 202217876738
(43) Date of publication of application: 10.07.2024
(73) Proprietor: Drägerwerk AG & Co. KGaA, 23558 Lübeck (DE)
(72) Inventor: WANG, Xianju, Bedford, Massachusetts 01730 (US); ZHANG, Zhe, Westford, Massachusetts 01886 (US)
(74) Representative: Haseltine Lake Kempner LLP
(86) International application number: PCT/IB2022/057425
(87) International publication number: WO 2023/031706

(56) References cited:
- CN-A- 110 179 456
- US-A1- 2013 338 519
- US-A1- 2020 093 389
- US-A1- 2021 052 180
- ZHANG QIFEI ET AL: "A Cascaded Convolutional Neural Network for Assessing Signal Quality of Dynamic ECG", COMPUTATIONAL AND MATHEMATICAL METHODS IN MEDICINE, vol. 2019, 20 October 2019 (2019-10-20), pages 1 - 12, XP055846197, ISSN: 1748-670X, Retrieved from the Internet <URL:https://downloads.hindawi.com/journals/cmmm/2019/7095137.pdf> DOI: 10.1155/2019/7095137

## Description

### CROSS REFERENCE TO RELATED APPLICATIONS

The present application claims priority to and the benefit of U.S. Prov. Pat. App. Ser. No. 63/238,604, which was filed on August 30, 2021 and U.S. Non-Provisional Patent Application No. 17/876,738 filed July 29, 2022, for all purposes, including the right of priority.

### TECHNICAL FIELD

The present disclosure relates generally to the field of electrocardiogram ("ECG") signal analysis. More particularly, the present disclosure relates to ECG waveform analysis including artifact detection and QRS-complex identification.

### BACKGROUND

Electrocardiogram ("ECG") is commonly used to monitor patients' heart conditions as well as detecting or predicting cardiac events. In clinical settings, ECG signals are captured in waveforms and analyzed by physiological monitoring devices. The physiological monitoring devices identify QRS-complexes, the combination of three waves in the ECG waveform corresponding to the depolarization of the right and left ventricles and the contraction of large ventricular muscles. For a normal sinus rhythm, R-wave (a sharp upward deflection) in the QRS-complex with a large amplitude and a small width, is suitable for measuring heart rate and other cardiac conditions. Physiological monitoring devices further classify the detected QRS-complexes into different types, based on features extracted from the morphology of each QRS-complex.

In clinical settings, however, noise contamination caused by artifact signals adversely impacts the precision and accuracy in ECG signal analysis including QRS-complex identification and subsequent beat classification. For example, noise contamination impacts the accuracy of algorithms designed to detect several cardiac pathologies such as arrhythmias. As a result, a high rate of false arrhythmia alarms will lead to alarm fatigue, where clinical providers are potentially desensitized to frequent invalid or nonactionable alarms and therefore, silencing the alarms with a risk of missing genuine and critical alarms.

A variety of sources may cause artifact signals, including physiological artifacts caused by patients and non-physiological artifacts caused by electric circuitry in the physiological monitoring devices and/or other devices in the clinical environment. Thus, it is important for physiological monitoring devices to accurately detect artifact signals, identify and analyze the corrupted segments of ECG signals contaminated by artifacts.

US 2020/093389 A1 appears to disclose methods and systems for continuously monitoring a patient for cardiac electrical abnormalities including atrial fibrillation, asystole, ventricular fibrillation and tachycardia.

US 2013/338519 A1 appears to disclose an apparatus and method for determining a signal quality of an input signal representing a repetitious phenomena derived from at least one sensor connected to a patient. A detector receives the input signal and determines data representing the repetitious phenomena from the input signal for use in determining at least one patient parameter. A measurement processor is electrically coupled to the detector that determines a first signal quality value by identifying at least one feature of the repetitious phenomena data and compares the at least one feature of a first set of the determined repetitious phenomena data with a second set of the determined repetitious phenomena data to determine a feature variability value and using the feature variability value to determine a stability value representative of the quality of the input signal.

US 2021/052180 A1 appears to disclose a cardiac monitoring system comprising a processor to receive a segment of an electrocardiogram (ECG) signal of a patient, and a memory to store the segment of the ECG. The processor is configured to identify QRS complexes in the segment of the ECG signal, compare the QRS complexes in the segment to the other QRS complexes in the segment to identify a main template QRS complex, identify the QRS complexes in the segment that are similar to the main template, determine RR intervals between consecutive similar QRS complexes to calculate RR variability in the RR intervals, and detect atrial fibrillation (AF) in the segment when RR variability is greater than a threshold value.

ZHANG QIFEI ET AL: "A Cascaded Convolutional Neural Network for Assessing Signal Quality of Dynamic ECG", COMPUTATIONAL AND MATHEMATICAL METHODS IN MEDICINE, vol. 2019, 20 October 2019 appears to disclose a method of using a convolutional neural network to assess dynamic ECG signal quality, taking into account motion artifacts and myoelectrical noise.

CN 110 179 456 A appears to disclose a method and device for training an electrocardio noise recognition model, and a method and device for detecting electrocardio noise. The method for training the electrocardio noise recognition model comprises the following steps: acquiring electrocardio record data as training data of the electrocardio noise recognition model, wherein the electrocardio record data comprise clean-segment data containing no noise and noise-segment data containing noise; calculating electrocardio noise parameters of the training data; constructing a noise characteristic vector according to the electrocardio noise parameters; and inputting the noise characteristic vector into a neural network model for training so as to obtain the electrocardio noise recognition model. The electrocardio noise recognition model trained by the method can more quickly and accurately analyze whether each heart beat contains noise or not, and avoid the condition of erroneously judging the low signal-to-noise ratio caused by noise as arrhythmia.

### BRIEF DESCRIPTION

There exists a need for improved detection and analysis of ECG signals, for the physiological monitoring device to identify artifact signals and accordingly, analyze ECG signals in real-time even in the presence of artifacts. There also exists a need of identifying and analyzing QRS-complexes in real-time in the presence of artifacts, for heart rate calculation, beat classification as well as alarm generation.

To resolve at least one or more of the above problems and potentially other present or future problems, one aspect of the present invention relates to an apparatus for determining artifact signals from a plurality of sample signals collected during a pre-determined time window from at least one lead configured to be affixed to a patient, as defined in appended independent claim 1.

Another aspect of the present invention relates to a computer-implemented method for determining artifact signals from a plurality of sample signals collected during a pre-determined time window from at least one ECG lead configured to be affixed to a patient, as defined in appended independent claim 8.

One or more embodiments of the present disclosure provide but are not limited to the following advantages. A variety of artifact signals that cause disturbance in ECG monitoring can be detected in real-time. For example, the embodiments in the present disclosure are capable of detecting physiological artifacts caused by patient motion, including motions associated with the patient's medical conditions (e.g., tremors, shivering) and regular muscular activities (e.g., brushing, combing). Additionally, the embodiments in the present disclosure are capable of detecting non-physiological artifacts including electromagnetic interference caused by physiological monitoring systems or other electrical devices in the clinical environment, as well as artifacts caused by cable and/or electrode malfunction. Thus, the embodiments in the present disclosure prevent artifact signals from being falsely identified as part of a QRS-complex, thereby increasing the accuracy in QRS-complex identification and classification, as well as the accuracy in heart rate calculation and alarm generation.

On the other hand, when a patient has medical conditions, the morphologies of the monitored ECG waveforms can be complex or irregular, and thus, difficult to differentiate from artifact signals. The embodiments in the present disclosure provide validation processes for identified artifact signals, thereby reducing the false-positive rate. With the complex or irregular ECG waveforms being accurately identified rather than treated as artifacts, the embodiments in the present disclosure are capable of analyzing different types of ECG waveforms accurately and generating alarms. Thus, clinical providers can promptly identify the medical conditions of the patient and provide treatment as needed, thereby improving clinical workflows.

### BRIEF DESCRIPTION OF THE DRAWINGS

In the drawings, like reference numbers generally indicate identical, functionally similar, and/or structurally similar elements.
FIG. 1 is a schematic diagram of an exemplary physiological monitoring system with a plurality of surface ECG leads connected to a patient, for analyzing ECG signals and detecting artifacts, according to one embodiment of the present disclosure;
FIG. 2 is a schematic diagram of an exemplary physiological monitoring device with a plurality of surface ECG leads connected to a patient, for analyzing ECG signals and detecting artifacts, according to one embodiment of the present disclosure;
FIG. 3 is a schematic diagram of another exemplary physiological monitoring system for analyzing ECG signals and detecting artifacts, according to one embodiment of the present disclosure;
FIG. 4 is a flow diagram of an exemplary process of analyzing ECG signals and detecting artifacts, according to one embodiment of the present disclosure;
FIG. 5A is a schematic diagram of an exemplary artifact signal detection process using an artificial neural network (ANN), according to one embodiment of the present disclosure;
FIG. 5B is a schematic diagram of another exemplary artifact signal detection process using an ANN according to one embodiment of the present disclosure;
FIG. 6A is an illustration of an exemplary sample signal recording from ECG leads for artifact signal detection using an ANN according to one embodiment of the present disclosure;
FIG. 6B is an illustration of another exemplary sample signal recording from ECG leads for artifact signal detection using an ANN according to one embodiment of the present disclosure;
FIG. 7A is a schematic diagram of an exemplary ECG signal analysis process for SQI calculation according to one embodiment of the present disclosure;
FIG. 7B is a schematic diagram of another exemplary ECG signal analysis process for SQI calculation according to one embodiment of the present disclosure;
FIG. 8 is a flow diagram of an exemplary process of artifact signal detection and ECG signal analysis according to one embodiment of the present disclosure;
FIG. 9A is an illustration of an exemplary sample signal recording from ECG leads before the QRS-complex validation process according to one embodiment of the present disclosure; and
FIG. 9B is an illustration of an exemplary sample signal recording from ECG leads after the QRS-complex validation process according to one embodiment of the present disclosure.

### DETAILED DESCRIPTION

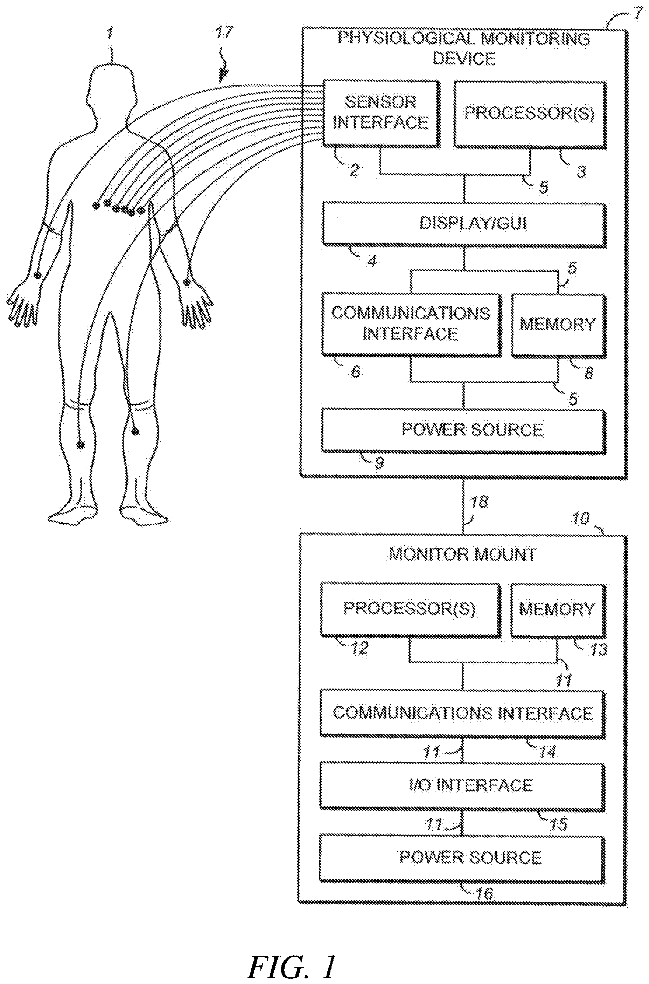

FIG. 1 is a schematic diagram of an exemplary physiological monitoring system with a plurality of surface ECG leads connected to a patient, for analyzing ECG signals and detecting artifacts, according to one embodiment of the present disclosure. As illustrated, the system includes a physiological monitoring device 7 capable of receiving physiological data from various sensors 17 connected to a patient 1, and a monitor mount 10 to which the physiological monitoring device 7 is removably mounted or docked.

The physiological monitoring device 7 is, for example, a patient monitor implemented to monitor various physiological parameters of patient 1 via sensors 17. The physiological monitoring device 7 includes a sensor interface 2, one or more processors 3, a display/graphical user interface ("GUI") 4, a communications interface 6, a memory 8, and a power source 9. The sensor interface 2 can be implemented in software or hardware and used to connect via wired and/or wireless connections to one or more physiological sensors and/or medical devices 17 for gathering physiological data from patient 1. The data signals from the sensors 17 include, for example, data related to an electrocardiogram ("ECG"), non-invasive peripheral oxygen saturation (SpO2), non-invasive blood pressure ("NIBP"), temperature, and/or tidal carbon dioxide (etCO2), apnea detection, and other similar physiological data.

The communications interface 6 allows the physiological monitoring device 7 to directly or indirectly (via, for example, the monitor mount 10) to communicate with one or more computing networks and devices. The communications interface 6 can include various network cards, interfaces, or circuitry to enable wired and wireless communications with such computing networks and devices. The communications interface 6 can also be used to implement, for example, a BLUETOOTH^{®} connection, a cellular network connection, and/or a WIFI^{®} connection. Other wireless communication connections implemented using the communications interface 6 include wireless connections that operate in accordance with but are not limited to, IEEE802.11 protocol, a Radio Frequency for Consumer Electronics (RF4CE) protocol, ZIGBEE^{®} protocol, and/or IEEE802.15.4 protocol.

Additionally, the communications interface 6 can enable direct (e.g., device-to-device) communications (e.g., messaging, signal exchange, etc.) such as from the monitor mount 10 to the physiological monitoring device 7 using, for example, a Universal Serial Bus ("USB") connection. The communications interface 6 can also enable direct device-to-device connection to other devices such as to a tablet, personal computer ("PC"), or similar electronic device; or to an external storage device or memory.

Power source 9 can include a self-contained power source such as a battery pack and/or include an interface to be powered through an electrical outlet (either directly or by way of the monitor mount 10). The power source 9 can also be a rechargeable battery that can be detached allowing for replacement. In the case of a rechargeable battery, a small built-in backup battery (or supercapacitor) can be provided for continuous power to be provided to the physiological monitoring device 7 during battery replacement. Communications between the components of the physiological monitoring device 7 (e.g., 2, 3, 4, 6, 8, and 9) are established using an internal bus 5.

As illustrated in FIG. 1, the physiological monitoring device 7 is connected to the monitor mount 10 via a connection 18 that establishes a communication connection between, for example, the respective communications interfaces 6, 14 of the devices 7, 10. Connection 18 enables the monitor mount 10 to detachably secure the physiological monitoring device 7 to the monitor mount 10. In this regard, "detachably secure" means that the monitor mount 10 can secure the physiological monitoring device 7, but the physiological monitoring device 7 can be removed or undocked from the monitor mount 10 by a user when desired. The connection 18 may include but is not limited to, a universal serial bus (USB) connection, parallel connection, a serial connection, a coaxial connection, a High-Definition Multimedia Interface ("HDMI") connection, or other similar connection known in the art connecting to electronic devices.

The monitor mount 10 includes processor(s) 12, a memory 13, a communications interface 14, an I/O interface 15, and a power source 16. Processor(s) 12 are used for controlling the general operations of the monitor mount 10. Memory 13 can be used to store any type of instructions associated with algorithms, processes, or operations for controlling the general functions and operations of the monitor mount 10.

The communications interface 14 allows the monitor mount 10 to communicate with one or more computing networks and devices (e.g., the physiological monitoring device 7). The communications interface 14 can include various network cards, interfaces, or circuitry to enable wired and wireless communications with such computing networks and devices. The communications interface 14 can also be used to implement, for example, a BLUETOOTH^{®} connection, a cellular network connection, and a WIFI^{®} connection. Other wireless communication connections implemented using the communications interface 14 include wireless connections that operate in accordance with, but are not limited to, IEEE 802.11 protocol, a Radio Frequency For Consumer Electronics (RF4CE) protocol, ZIGBEE^{®} protocol, and/or IEEE 802.15.4 protocol.

The communications interface 14 can also enable direct (e.g., device-to-device) communications (e.g., messaging, signal exchange, etc.) such as from the monitor mount 10 to the physiological monitoring device 7 using, for example, a USB connection, coaxial connection, or other similar electrical connection. The communications interface 14 can enable direct (e.g., device-to-device) to another device such as to a tablet, PC, or similar electronic device; or an external storage device or memory.

The I/O interface 15 can be an interface for enabling the transfer of information between monitor mount 10, one or more physiological monitoring devices 7, and external devices such as peripherals connected to the monitor mount 10 that need special communication links for interfacing with processor(s) 12. The I/O interface 15 can be implemented to accommodate various connections to the monitor mount 10 that include but is not limited to, a universal serial bus ("USB") connection, parallel connection, a serial connection, coaxial connection, a High-Definition Multimedia Interface ("HDMI") connection, or other known connection in the art connecting to external devices.

Power source 16 can include a self-contained power source such as a battery pack and/or include an interface to be powered through an electrical outlet (either directly or by way of the physiological monitoring device 7). The power source 16 can also be a rechargeable battery that can be detached allowing for replacement. Communications between the components of the monitor mount 10 (e.g., 12, 13, 14, 15, and 16) are established using an internal bus 11.

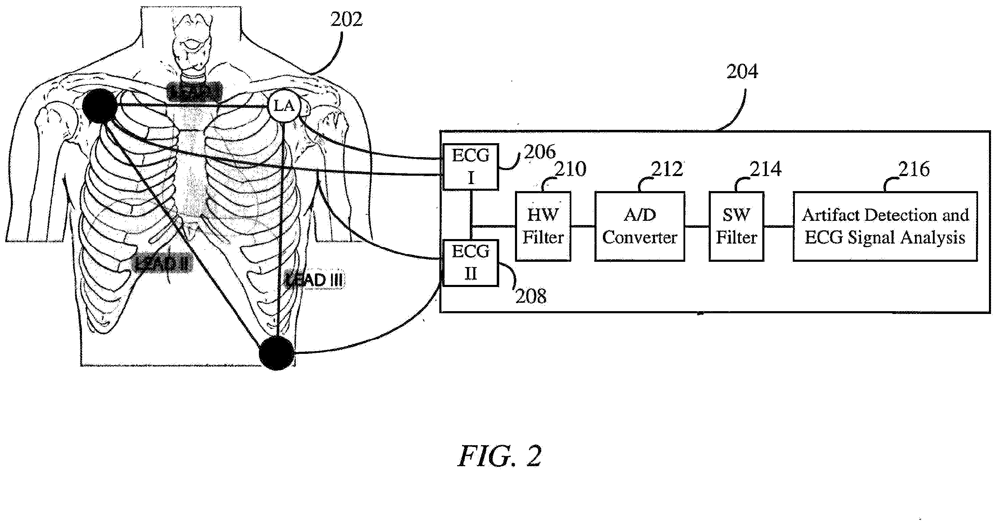

FIG. 2 is a schematic diagram of an exemplary physiological monitoring device with a plurality of surface ECG leads connected to a patient, for analyzing ECG signals and detecting artifacts, according to one embodiment of the present disclosure. A plurality of ECG electrodes is attached to pre-determined positions on the body of a patient 202 (e.g., LA = Left Arm, RA = Right Arm, LL = Left Leg, as illustrated in FIG. 2). As illustrated, two electrodes attached to the patient's left arm (LA) and right arm (RA), respectively, are connected to a physiological monitoring device 204 via the sensor interface 206 and configured to measure electrical potential signals of the heart from a first vector (e.g., ECG I 206). Similarly, two electrodes attached to the right arm (RA) and left leg (LL), respectively, are connected to the physiological monitoring device 204 via the sensor interface 208 for ECG signal measurements from a second vector (e.g., ECG II 208). The signals from the two ECG leads are filtered using hardware filters 210 (e.g., high-pass filters and/or low-pass filters), converted to digital forms using A/D converter 212, and further filtered using software filtering strategies 214 (e.g., low-pass filters). The sampling rate for the conversion may be in the range of 100 - 1,000 samples per second (sps), for example, 120 - 750 sps, 200 - 500 sps, or 150 - 350 sps. Subsequently, the digital data stream is passed for ECG signal analysis 216, which will be described in more detail as follows.

It should be noted that the present disclosure is not limited to the numbers and types of ECG leads used for collecting ECG signals and subsequent analysis. In other words, ECG signals may be received from a single ECG lead, two ECG leads, or more. Additionally, the two or more leads are not limited to ECG I (from electrodes attached to the patient's left arm and right arm) and ECG II (electrodes attached to the patient's right arm and left leg) as illustrated in FIG. 2. Commonly used ECG configurations include 3-lead, 5-lead, 6-lead, 12-lead. In this way, the overall magnitude and direction of the heart's electrical activity are captured throughout the heartbeat. Depending on the need of the patient (e.g., medical condition of the patient, location of the patient, ECG monitoring device the patient is connected to, and/or medical procedure the patient is applied to), different lead configurations may be used. Accordingly, the present disclosure is adapted to receive and analyze ECG signals from different leads and detect artifact signals as described below.

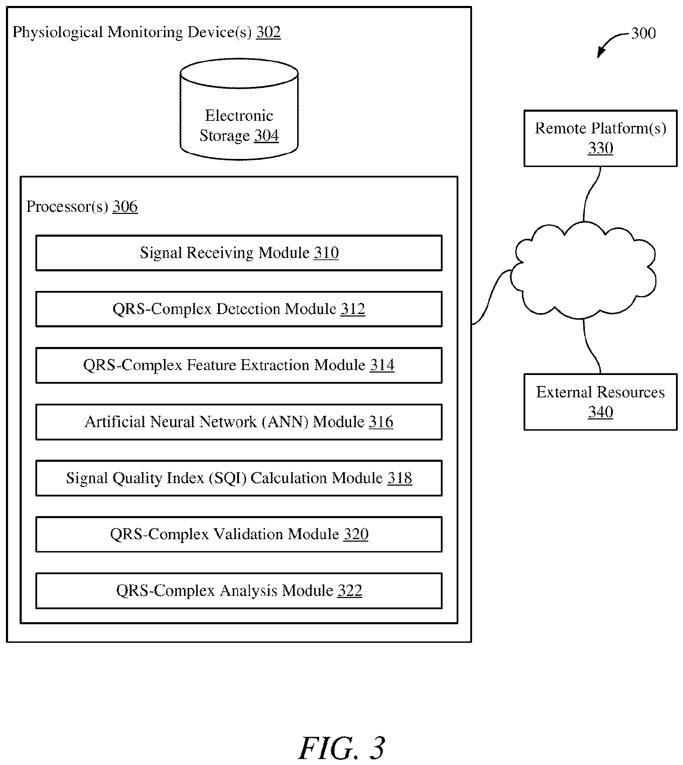

FIG. 3 is a schematic diagram of another exemplary physiological monitoring system for analyzing ECG signals and detecting artifacts, according to one embodiment of the present disclosure. As illustrated, system 300 may include one or more components, for example, a physiological monitoring device 302 in wired or wireless connection with external resources 340 and remote platform(s) 330. In one embodiment, physiological monitoring device 302, remote platform(s) 330, and/or external resources 340 may be operatively linked via one or more electronic communication links. Such electronic communication links may be established, at least in part, via a network such as the Internet and/or other networks or other communication media. Remote platform(s) 330 and external resources 340 may include medical devices (e.g., a physiological monitoring device, a therapy device, a diagnostic device), one or more servers, a desktop computer, a laptop computer, a handheld computer, a tablet computing platform, a NetBook, a smartphone, and/or other platforms. For example, the physiological monitoring device 302 may receive ECG signals, detect artifact signals and identify QRS-complexes. The analyzed ECG signals may be transmitted and stored in remote platform(s) 330 and external resources 340, for example, in nurse station or patient's electronic medical record system. The monitoring device 302 may also generate alarms based on the detected ECG signals and transmit the alarms to remote platform(s) 330 and external resources 340, for example, the hospital's alarm output system.

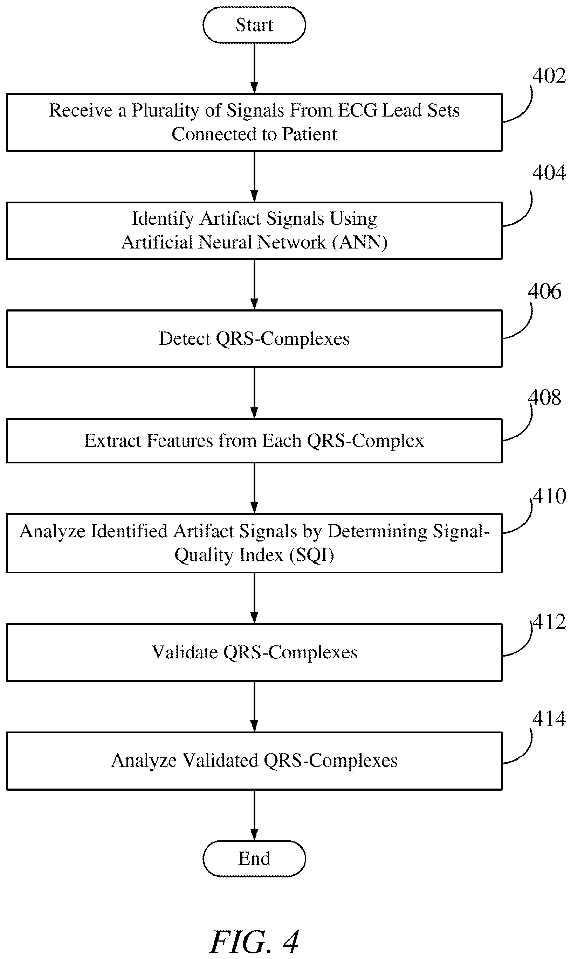

One or more components of the system 300, e.g., physiological monitoring device 302 may be the same or substantially similar as physiological monitoring device 7 illustrated in FIG. 1 and device 204 illustrated in FIG. 2. Physiological monitoring device 302 is capable of receiving physiological data (e.g., ECG signals) from a plurality of ECG leads affixed to the patient. Processor(s) 306 may be configured to analyze ECG signals in accordance with FIG. 4, which illustrates an exemplary process of ECG signal analysis and artifact signal detection. For example, processor(s) 306 may execute signal receiving module 310 and perform the signal receiving process 402 as illustrated in FIG. 4. The received analog signals may further be filtered via hardware and software filters and converted into a digital data stream via an A/D converter, in accordance with the embodiments illustrated in FIG. 2.

Processor(s) 306 may further execute QRS-complex detection module 312 and QRS-complex feature extraction module 314, in accordance with steps 406 and 408 as illustrated in FIG. 4, respectively. A QRS-complex is commonly the central and most visually obvious part of an ECG waveform, with a duration of approximately 80 milliseconds (ms) - 100 ms in adults. Processor(s) 306 may identify the QRS-complex by, for example, identifying an R-wave within the QRS-complex. Processor(s) 306 may search one or more edge points of the received sample signals, including a starting point, a peak point, a tail point, and an endpoint. By defining one or more edge points, processor(s) 306 may identify the R-wave and its corresponding QRS-complex.

Concurrently or subsequently, processor(s) 306 may further extract one or more features from the identified QRS-complexes, including but not limited to amplitude, width, morphology, curvature, symmetry, peak direction, intervals of different waves including R-R intervals (i.e., the time interval between two consecutive R-waves) and P-R intervals (time interval between the beginning of the upslope of the P wave to the beginning of QRS wave). Based on these extracted features, processor(s) 306 may further classify the QRS-complexes into different types, including a normal beat or a bundle branch block beat (N), a ventricular ectopic beat (V), a supraventricular ectopic beat (S), a fusion of ventricular and normal beat (F) and a paced beat or a beat that cannot be classified (Q). Each beat type has its characteristic features and accordingly, physiological monitoring device 302 may store in electronic storage 304 a QRS-complex template database including a pre-determined threshold value or a range of pre-determined threshold values for each feature of the classified beat type. When a new QRS-complex is identified, processor(s) 306 may extract one or more features and compare them with the pre-determined threshold value or a range of threshold values, thereby classifying the QRS-complex into a specific beat type based on the comparison results. In another embodiment, the pre-determined threshold values or the range of threshold values may be dynamically updated. That is, after a new beat is classified, the extracted features of this beat are used to update the existing QRS-complex template database. When the identified QRS-complex and its corresponding classification indicate cardiac conditions, processor(s) 306 may generate alarms and display one or more extracted features for clinical providers. For example, the duration, amplitude, and morphology of the QRS-complex are useful for clinical providers to diagnose cardiac arrhythmias, conduction abnormalities, ventricular hypertrophy, myocardial infarction, electrolyte derangements, and other cardiac conditions.

In clinical settings, artifact signals are often mixed with ECG signals, which makes it a challenge to the QRS-complex identification, feature extraction, and subsequently, beat classification. For example, an artifact signal with a high amplitude and narrow width (e.g., high-frequency artifacts and low-frequency artifacts) may adversely impact the identification of R-waves and subsequent feature extraction (e.g., R-R interval, P-R interval, R-wave amplitude). On the other hand, baseline shift artifacts may corrupt the ECG signals and interfere with the identification of ST segments, which are important diagnostic markers for ischemia and infarction. The present disclosure provides the detection of artifact signals and differentiating artifacts from QRS-complexes. Thus, it avoids disturbance caused by various types of and often unexpected or random artifact signals captured by the monitoring device.

In the context of the claimed invention, processor(s) 306 is configured to execute artificial neural network ("ANN") module 316 and identify artifact signals in accordance with step 404 as illustrated in FIG. 4. The ANN is based on one or more interconnected groups of artificial neurons, where each artificial neuron receives one or more input, applies transforms to each input via activation functions, generates an output, and transmits it to other artificial neurons. The transmitted output signal will be used by other artificial neurons as one of their corresponding input, transformed by applying activation functions, and generate another output.

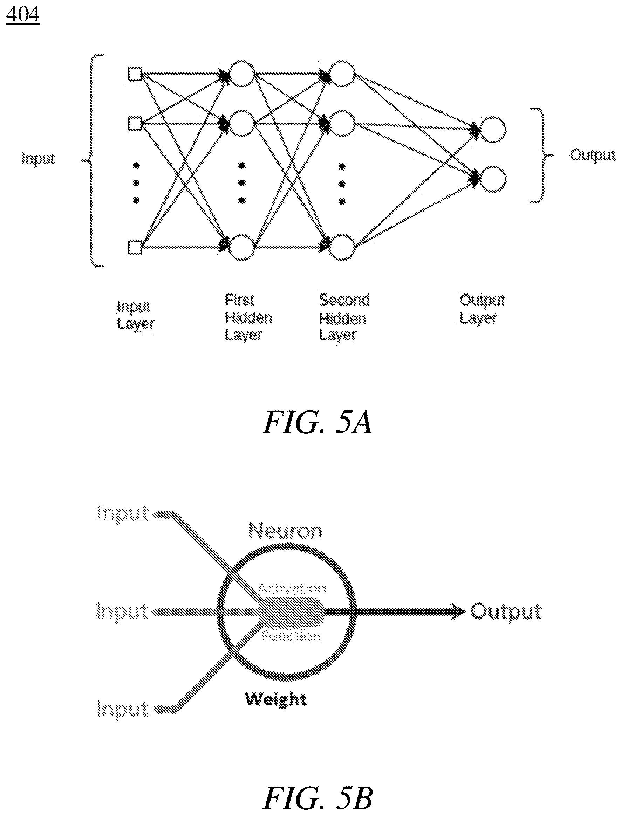

FIG. 5A is a schematic diagram of an exemplary artifact signal detection process using ANN, according to one embodiment of the present disclosure. As illustrated, the ANN is aggregated into multiple layers including an input layer, one or more hidden layers (e.g., first hidden layer and second hidden layer in FIG. 5A), and an output layer. In one embodiment, sample signals collected during a pre-determined time window from an ECG lead affixed to the patient may be fed into the input layer. The sample signals that are fed into the input layer may include all sample points (each with an amplitude) collected during the pre-determined time window, for example, 100 ms - 10 seconds (s), 200 ms - 7 s, 500 ms - 5 s. Alternatively, the sample signals fed into the input layer may include selected sample points with an amplitude exceeding a pre-determined threshold value or a range of pre-determined threshold values. The selected sample points may include one or more of a minimum sample point (i.e., the sample point with a minimum amplitude), a maximum sample point (i.e., the sample point with a maximum amplitude), and a medium sample point (i.e., the sample point with an intermediate amplitude between a maximum and a minimum amplitude). The one or more extracted features that are fed into the input layer may include but are not limited to a difference in amplitude between maximum and minimum sample points, a difference in amplitude between consecutive sample points, sum (or normalized sum) of amplitudes of selected sample points, a standard deviation in amplitudes of selected sample points or a sum (or a normalized sum) of the standard deviations. The extracted features may also include the number of identified edge signals, where each edge signal is determined when a difference between two consecutive sample points exceeds a pre-determined threshold. The extracted features may also include a sum (or normalized sum) of amplitudes of all of the identified edge signals.

As further illustrated in FIG. 5A, each hidden layer may include a plurality of artificial neurons. For example, each input within the input layer may be fed into each artificial neuron within the first hidden layer, where each neuron generates an output to be fed into a plurality of artificial neurons within the second hidden layer, and so on. Alternatively or additionally, an output generated from the second hidden layer may be sent back to the first hidden layer for further calculation. It should also be noted that the present disclosure does not intend to limit the number of input data, hidden layers, artificial neurons within each hidden layer, or the connections (or the directions of the connections) among different layers.

FIG. 5B is a schematic diagram of another exemplary artifact signal detection process using an ANN according to one embodiment of the present disclosure. As illustrated, each artificial neuron receives a plurality of input and generates one output based on a transformation (e.g., an activation function) of each input. The activation function may include but is not limited to non-linear functions (e.g., Relu function, Sigmoid function, Tanh function, and Softmax function), as well as linear functions. Each artificial neuron within different hidden layers may use different activation functions. For example, a first hidden layer may use the Sigmoid function and the second hidden layer may use the Softmax function. All artificial neurons within the same hidden layer may use the same activation function.

In one embodiment, a weight factor (W) and/or a bias factor (B) may also be used. A weight factor controls the strength of the connection between two artificial neurons. In other words, a weight factor decides how much influence the input will have on its corresponding output. When a weight factor is applied in an activation function, it manipulates the shape and/or curvature of the activation function. A bias factor is an additional input used in the activation function and it shifts the activation function along its input axle without changing the shape or curvature of the function. Each input may have an associated weight factor and each neuron may have an associated bias factor. For example, there are N neurons (N is an integer, N ≥ 2) in one hidden layer and there are M (M is an integer, M ≥ 2) inputs that are fed into each of the N neurons to generate an output. Each of the N inputs may have its associated weight factor W (W₁, W₂, ..., W_{N}), and each of the M neurons may have its associated bias factor B (B₁, B₂, ..., B_{M}). Each of the weight factors W and bias factors B may adjust as the learning proceeds. The initial weight factor W₁ and initial bias factor B₁ may be pre-determined values obtained from a training process using an existing ECG waveform database. Alternatively, each of the weight factors W and bias factors B may be pre-determined values obtained from a training process using an existing ECG waveform database.

The output generated from the last hidden layer (e.g., the second hidden layer illustrated in FIG. 5A) may be fed into the output layer and eventually generate two outputs. In one embodiment, when the processor(s) collect sample signals over a pre-determined time window from an ECG lead, select sample points from which extract a plurality of features, these features are used as input of the ANN. The generated output indicates the probability of the existence of artifact signals within the collected sample signals over the determined time window. For example, the two outputs may include p_{clean} and pₙₒᵢₛₑ (p_{clean} + pₙₒᵢₛₑ = 1), where p_{clean} indicates a low probability of the existence of artifacts in the sample signals, and pₙₒᵢₛₑ indicates a high probability of the existence of artifacts. When at least one of p_{clean}, pnoise or a difference between p_{clean} and pₙₒᵢₛₑ (e.g., pnoise - p_{clean}) exceeds a pre-determined threshold, the processor(s) may determine that the collected sample signals include artifacts.

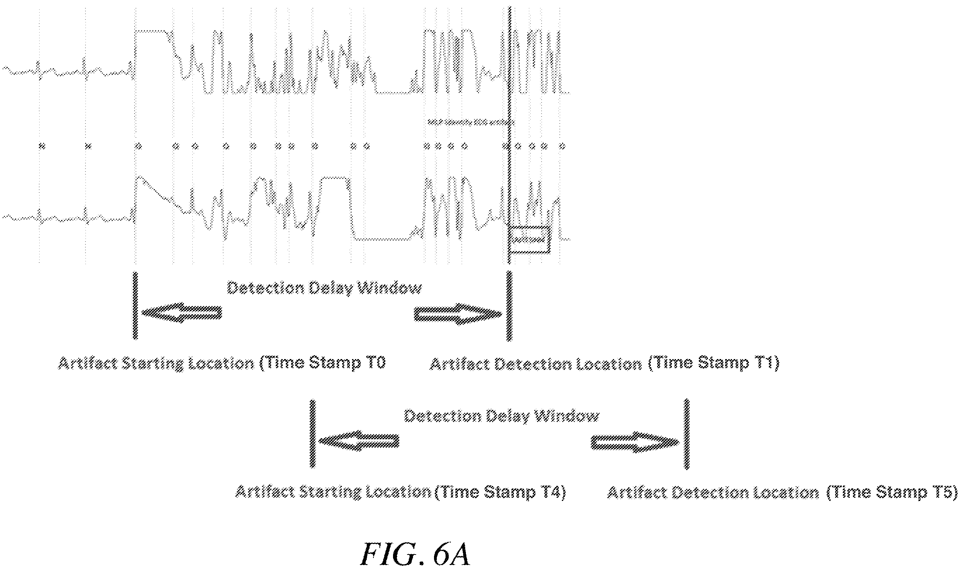

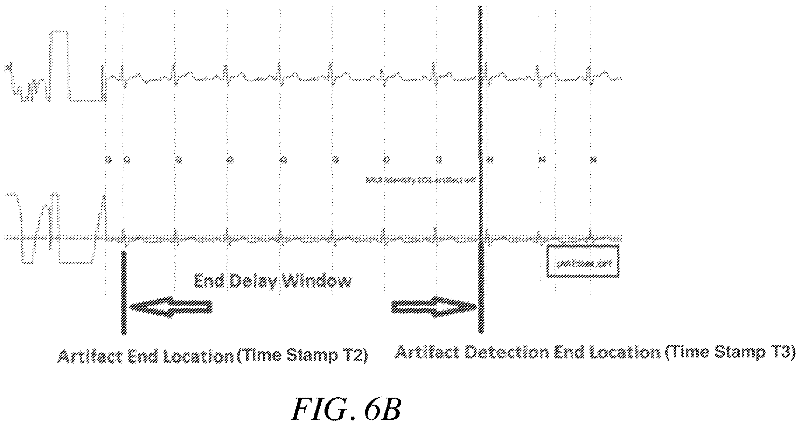

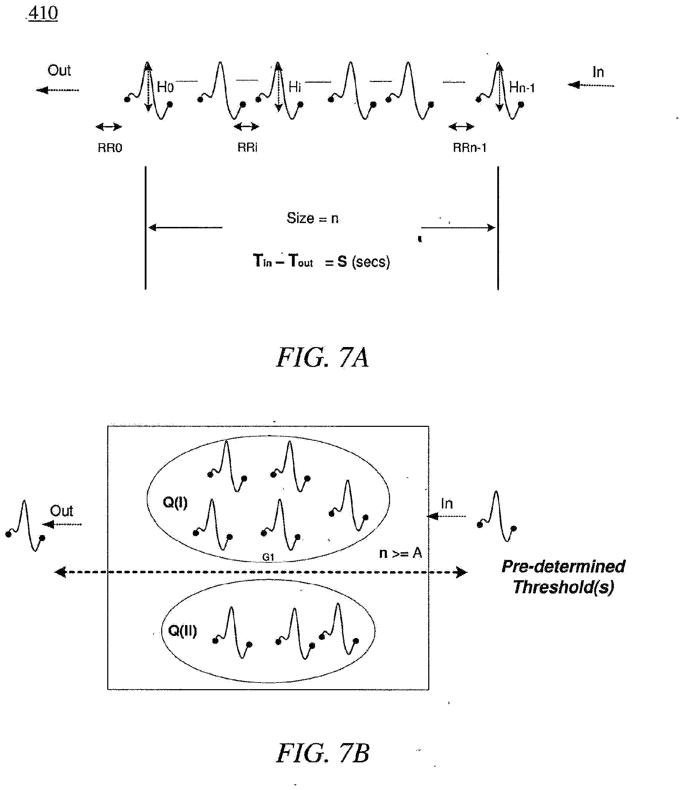

FIGs. 6A and 6B are illustrations of exemplary sample signal recordings from ECG leads for artifact signal detection using ANN according to one embodiment of the present disclosure. Sample signals are continuously collected from two ECG leads connected to a patient. As illustrated in FIG. 6A, for each ECG lead, sample signals including artifacts are collected and recorded from timestamp T0, and the processor(s) start executing step 404 at timestamp T1 and identify whether the collected sample signals during a detection window (from T0 to T1) include artifact signals. For example, when the difference between pnoise and p_{clean} (e.g., pnoise - p_{clean}) exceeds a pre-determined threshold, the sample signals during the detection window (from T0 to T1) are deemed to include artifacts. As the physiological medical device continuously acquires sample signals from the ECG leads, the processor(s) keep executing step 404 and determine whether the collected sample signals during a preceding detection window include artifact signals. In one embodiment, only when two consecutive detection windows are analyzed, via ANN process, to include low or minimum artifacts, the processor(s) determine that the collected sample signals within these two windows are "good". If one detection window with "good" sample signals is followed by a subsequent detection window including substantial artifact or vice versa, the processor(s) will further analyze the sample signals in both windows, for example, by calculating SQI values as described in FIGs. 7A-7B.

Optionally, the plurality of detection windows may overlap. As illustrated in FIG. 7A, for example, instead of consecutive detection windows with no overlap, an earlier window (e.g., from T0 to T1) may be partially overlapped with a subsequent window (e.g., from T4 to T5, where T0 < T4 < T1, and T5 > T1), and so on. Thus, sample signals collected within the detection windows from T0 to T1 and from T4 to T5 are analyzed, respectively, where the sample signals within each overlapped window (e.g., from T4 to T1) are analyzed twice. When the probability results from ANN processes indicate low or minimum artifacts within both detection windows, the sample signals within the overlapped window are identified as "good". As such, the accuracy of artifact signal identification is further improved.

As illustrated in FIG. 6B, the sample signals collected during a previous detection window that ends at the timestamp T2 are deemed to include artifacts, the processor(s) keep performing the ANN process by executing step 404 and analyze sample signals in the detection window from T2 to T3. To ensure the accuracy in identifying artifact signals, in one embodiment, when a preceding detection window is identified to include artifacts, the processor(s) keep analyzing the sample signals collected in the following at least two consecutive detection windows. As illustrated, for example, the duration between the timestamps T2 and T3 may be twice the duration of a typical detection window. Only when the sample signals in both windows are deemed to include low or minimum artifacts via ANN process, the processor(s) may confirm the sample signals include low or minimum artifacts (illustrated as ARTSNN_OFF in FIG. 6B).

In clinical settings, the differentiation between an artifact signal and ECG signals can be challenging, because patients' cardiac conditions may cause uncommon or irregular morphologies in the QRS-complexes that can be falsely identified as an artifact. One example is premature ventricular beat (PVC). PVC originates in the left ventricle when it has a positive deflection causing an R-wave with large amplitude or a negative deflection causing a deep S-wave. Further, the QRS-complex of a PVC is widened, as the P wave is not conducted and is often notched. Accordingly, when sample signals collected during a detection window are identified to include artifact signals, the processor(s) may further validate these signals and ensure QRS-complexes with particular morphologies are not falsely identified as artifacts. In one embodiment, the processor(s) execute SQI calculation module 318 in accordance with step 410 as illustrated in FIG. 4. When a calculated SQI exceeds a pre-determined threshold, these sample signals are validated as acceptable ECG signals, despite ANN analysis identifies them to include artifacts. Thus, the embodiments of the present disclosure further improve accuracy in identifying and differentiating artifact signals from QRS-complexes with uncommon morphologies.

FIG. 7A illustrates an exemplary ECG signal analysis process for SQI calculation according to one embodiment of the present disclosure. As illustrated, the processor(s) continuously collect sample signals during a pre-determined time window, identify a plurality of QRS-complexes, and extract features from each QRS-complex. For example, there are n QRS-complexes within a pre-determined time window with a duration of S seconds. The processor(s) may calculate an SQI value corresponding to this time window by comparing the similarities in the extracted features of the QRS-complexes. The extracted features used to calculate SQI include but are not limited to an amplitude of an R-peak, a width of an R-peak, an R-R interval, an area of QRS-complex, and an area of an S region (as called S-wave area). The duration of the pre-determined time window (S) may be in the range of 0.5 - 30 s, 1 - 20 s, 5 - 15 s, and so on. The number of QRS-complexes (n) may vary accordingly based on the duration of the time window and cardiac conditions of the patient. In one embodiment, SQI is calculated using a probability density function with amplitude Hᵢ (0 ≤ i ≤ n-1) and R-R interval RRᵢ (0 ≤ i ≤ n-1) as variables. When the calculated SQI exceeds a pre-determined threshold, the sample signals collected within this pre-determined time window are validated as "good" signals with low or minimum artifacts. A low SQI value may indicate the collected sample signals are noisy. To further improving the efficiency in artifact detection, in another embodiment, the QRS-complexes may be updated in real-time may follow a "first-in-first-out" rule. That is, the duration of the time window (S) and/or the number of QRS-complexes within the time window (n) is fixed. When a new QRS-complex is identified, the QRS-complex collected at the earliest time will be removed.

To further increase the efficiency in calculating SQI while maintaining accuracy, the processor(s) may select QRS-complexes within the pre-determined time window for SQI calculation. FIG. 7B illustrates an exemplary ECG signal analysis process for SQI calculation according to one embodiment of the present disclosure. Instead of using all of the identified QRS-complexes within the time window, the processor(s) may cluster the QRS-complexes into at least two clusters (e.g., clusters Q(I) and Q(II) illustrated in FIG. 7B), using one or more pre-determined thresholds. For example, an amplitude threshold may be used, where all QRS-complexes having an amplitude exceeding the threshold are clustered in Q(I) and otherwise, in Q(II). Additionally or alternatively, the size of the clusters may exceed a pre-determined threshold, that is, the number of the QRS-complexes (n) within the cluster (e.g., Q(I)) may exceed a pre-determined threshold (A), to be used for SQI calculation. Thus, the processor(s) may select the cluster with a larger number of QRS-complexes for the SQI calculation.

As described above, when the calculated SQI corresponding to the pre-determined time window exceeds a pre-determined threshold, the collected sample signals within the time window are deemed as "good" ECG signals and all of the identified QRS-complexes could potentially be used for subsequent beat classification and QRS-complex template database update. On the other hand, even though sample signals collected within a pre-determined time window are determined as noisy signals including artifacts via ANN analysis and SQI calculation, the processor(s) further analyze and validate the QRS-complexes identified within the time window. In one embodiment, processor(s) 306 may execute QRS-complex Validation Module 320 in accordance with step 412 as illustrated in FIG. 4, and QRS-complex Analysis Module 322 in accordance with step 414. As such, even when the ANN analysis and subsequent SQI calculation determined a segment of sample signals in a pre-determined time window as noisy signals, the QRS-complexes can still be accurately identified and differentiated from artifact signals.

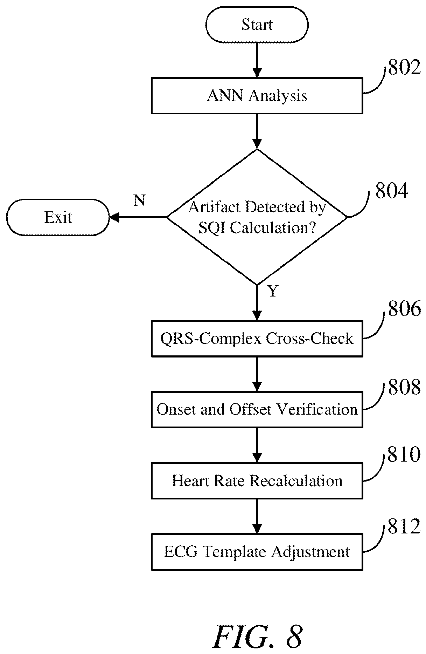

As illustrated in step 802 of FIG. 8, the processor(s) is configured to determine whether a segment of sample signals collected from ECG leads within a pre-determined time window includes artifacts via ANN (see module 316 of FIG. 3 in accordance with step 404 as illustrated in FIG. 4). In the case that the ANN analysis deemed the sample signals to include artifacts, the processor(s) is configured to further calculate SQI of the segment of the sample signal (step 804, as see module 318 of FIG. 3 in accordance with step 410 as illustrated in FIG. 4). When the SQI exceeds a pre-determined threshold indicating a substantial similarity among QRS-complexes within the time window with low or minimum artifacts, ANN determination will be discarded and superseded by the SQI analysis indicating "good" signals ("N" in step 804). When the calculated SQI fails to exceed the pre-determined threshold, the sample signals are confirmed to include artifacts ("Y" in step 804). Processor(s) 316 further validates the identified QRS-complexes by cross-checking between multiple ECG leads (step 806) and onset and offset verification (step 807). Concurrently or subsequently, the processor(s) may further analyze the validated QRS-complexes by e.g., recalculating heart rate (step 810) and adjust the ECG template database (step 812).

It should be noted that even when the probability results from the ANN process indicate the sample signals as "good", these sample signals could still be analyzed for SQI calculation, thereby ensuring the accuracy in identifying artifacts. Although not illustrated, in another embodiment, to improve efficiency in data processing, when the probability results from the ANN process indicate the sample signals as "good", the processor(s) may not need to further analyze the artifacts by calculating SQI.

When the sample signals are confirmed to include artifacts via step 804, the processor(s) further perform step 806 and validate the identified QRS-complexes by cross-checking between multiple ECG leads. To be specific, for each ECG lead, the processor(s) determine one or more fiducial points (e.g., onset point, offset point, and peak point) included in each identified QRS-complex and compare the corresponding fiducial points on different leads. For example, the processor(s) may determine and compare the fiducial point of the R-peak in the same QRS-complex measured from lead I (*f_{I}*) and lead II (*f_{II}*), respectively. When an absolute value of a difference between the two fiducial points (|*f_{I} - f_{II}*|) is within a pre-determined threshold, the corresponding QRS-complex is confirmed as a validated QRS-complex. Optionally, other fiducial points (e.g., onset/offset points corresponding to the same QRS-complex measured from leads I and II) may be compared to validate QRS-complexes, thereby avoiding QRS-complexes from being mistakenly identified as artifacts.

Additionally or alternatively, for each ECG lead, the processor(s) may also perform step 808 and validate the QRS-complexes by verifying onset and offset fiducial points. For example, the processor(s) may determine the onset and offset points of the R-wave in the same QRS-complex measured from lead I (on*_{I}* and off*_{I}*) and lead II (on*_{II}* and off*_{II}*), respectively. Based on the onset and offset points, the processor(s) may determine the area of the R-wave from the corresponding ECG lead and compare the extent of an area overlap on different ECG leads. When the extent of the area overlap exceeds a pre-determined threshold, the corresponding QRS-complex may be confirmed as a validated QRS-complex.

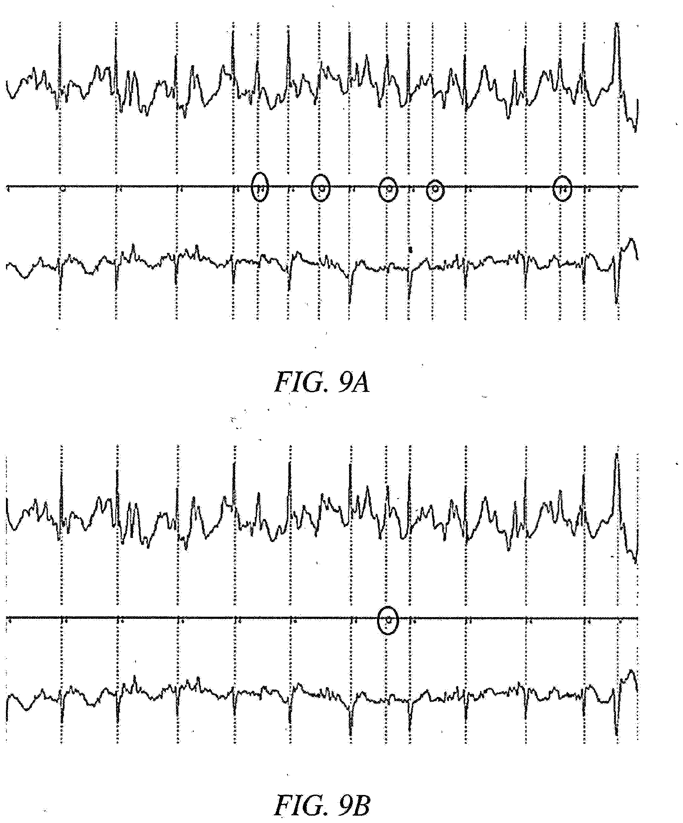

FIGs. 9A and 9B illustrate exemplary sample signal recordings from ECG leads before and after the QRS-complex validation process, respectively, according to one embodiment of the present disclosure. As shown in FIG. 9A, a segment of sample signals within a pre-determined time window records a plurality of QRS-complex identified as normal beats (N) and unclassified beats (Q). The unclassified beats may be caused by artifact signals or specific cardiac conditions of a patient. The processor(s) may further perform the validation process for each beat. In accordance with FIG. 8, the validation process may remove falsely identified N beats and Q beats as illustrated in FIG. 9B, thereby improving the accuracy in QRS-complex identification in the presence of artifacts.

In another embodiment as illustrated in FIG. 8, the processor(s) may further analyze the validated QRS-complexes, including recalculating heart rate (step 810). When the cardiac rhythm is regular, heart rate (HR) can be determined based on the interval between two successive QRS-complexes (e.g., R-R intervals). Artifact signals, however, can be present between adjacent normal R peaks and falsely identified as another independent R peak. As a result, the calculated heart rate can be abnormally high. To increase the accuracy in heart rate calculation, the processor(s) may analyze the validated QRS-complexes and recalculate the heart rate. As such, even if the validated QRS-complexes include one or more artifact signals, the recalculation of the heart rate may minimize the impact caused by the artifacts. For example, the processor(s) may establish an HR buffer including a defined number of previously calculated HR values ranked in ascending order (i.e., HR₁, HR₂, ..., HRᵢ, HRᵢ₊₁, ..., HR_{N}, where HRᵢ ≤ HRᵢ₊₁, 1 ≤ i ≤ N - 1). The output of the heart rate may be calculated by, for example, averaging part or all of the HR values or selecting the medium HR value (i.e., HR_{N/2}). When a new HR value (HR_{new}) is calculated based on the validated QRS-complexes, the processor(s) may compare HR_{new} with the existing HR buffer and recalculate the output of the heart rate based on the ranking of the new HR value. For example, the processor(s) may compare HR_{new} with the medium HR value (i.e., HR_{N/2}). If HR_{new} is larger than HR_{N/2}, the output of the heart rate may be recalculated based on HR_{new} and at least one of HR_{N/2}, HR_{(N/2)+1}, HR_{(N/2)+2}, ..., HR_{N}. If HR_{new} is smaller than HR_{N/2}, the output of the heart rate may be recalculated based on HR_{new} and at least one of HR₁, HR₂, ..., HR_{N/2}.

In another embodiment as illustrated in FIG. 8, the processor(s) may further analyze the validated QRS-complexes, by managing the QRS-complex template database (step 812). For example, memory 8 (illustrated in FIG. 1) or electronic storage 302 (illustrated in FIG. 3) may store a QRS-complex template database containing basic features of QRS-complexes that are built up and updated real-time by adding newly identified and classified QRS-complexes. The accuracy of the template database significantly impacts the QRS-complex identification and classification. When artifacts are falsely identified as a part of a QRS-complex and included in the template database, such contamination may cause inaccurate identification of arrhythmia events and thus, a high rate of false arrhythmia alarms. To minimize the adverse impact of including artifacts into the template database, the processor(s) may further analyze the validated QRS-complexes and adjust the threshold for approving the validated QRS-complexes to be added into the template database. The processor(s) may extract one or more features from the validated QRS-complexes, including but not limited to R-R intervals, amplitude and/or width of R-wave, amplitude of P-wave, the polarity of ST-segment. The threshold for each feature may be adjusted to be different from the threshold values for approving the QRS-complexes identified from sample signals confirmed by ANN and/or SQI calculation as "good" signals. In one embodiment, the feature threshold used to select validated QRS-complexes from "noisy" sample signals is adjusted to be higher than those to select QRS-complexes identified from "good" signals. Only when the validated QRS-complexes have one or more features that are within the adjusted threshold, they can be approved to be added into the existing template database. As such, even if the validated QRS-complexes still include one or more artifacts, the further analysis of QRS-complexes and threshold adjustment may reduce the risk of including artifacts in the template data and therefore, improve the accuracy of QRS-complex identification and classification, as well as reduce false alarms.

It should be noted that one or more of the steps 402, 404, 406, 408, 410, 412, and 414 in FIG. 4 may be used, where the numbers and/or orders of the steps are for exemplary purposes, and not intended to limit the scope of the disclosure. For example, the QRS-complex detection (step 406) and feature extraction (step 408) can occur before or concurrently with the artifact signal detection using ANN (step 404). It should be noted that one or more of the steps 802, 804, 806, 808, 810, and 812 in FIG. 8 may be used, where the numbers and/or orders of the steps are for exemplary purposes, and not intended to limit the scope of the disclosure.

It is also contemplated that the implementation of the components of the present disclosure can be done with any newly arising technology that may replace any of the above implementation technologies.

In general, it is contemplated by the present disclosure that the physiological monitoring device and the monitor mount (e.g., device 7 and device mount 10 as illustrated in FIG. 1, device 204 as illustrated in FIG. 2 and device 302 as illustrated in FIG. 3) include electronic components or electronic computing devices operable to receive, transmit, process, store, and/or manage patient data and information associated performing the functions of the system, which encompasses any suitable processing device adapted to perform computing tasks consistent with the execution of computer-readable instructions stored in a memory or a computer-readable recording medium.

Further, any, all, or some of the computing devices in the physiological monitoring device and the monitor mount (e.g., device 7 and device mount 10 as illustrated in FIG. 1, device 204 as illustrated in FIG. 2 and device 302 as illustrated in FIG. 3) may be adapted to execute any operating system, including Linux, UNIX, Windows Server, etc., as well as virtual machines adapted to virtualize execution of a particular operating system, including customized and proprietary operating systems. The physiological monitoring device and the monitor mount are further equipped with components to facilitate communication with other computing devices over one or more network connections, which may include connections to local and wide area networks, wireless and wired networks, public and private networks, and any other communication network enabling communication in the system.

By way of example, hardware processors described in the present disclosure (e.g., processor(s) 3 and 12 as illustrated in FIG. 1 and processor(s) 306 as illustrated in FIG. 3) are used for controlling the general operations of a physiological monitoring device (e.g., device 7 as illustrated in FIG. 1, device 204 as illustrated in FIG. 2 and device 302 as illustrated in FIG. 3). Each one of the one or more processors can be, but are not limited to, a central processing unit ("CPU"), a hardware microprocessor, a multi-core processor, a single-core processor, a field-programmable gate array ("FPGA"), a microcontroller, an application-specific integrated circuit ("ASIC"), a digital signal processor ("DSP"), or other similar processing devices capable of executing any type of instructions, algorithms, or software for controlling the operation and performing the functions of the physiological monitoring device.

Hardware processors described in the present disclosure (e.g., processor(s) 3 and 12 as illustrated in FIG. 1 and processor(s) 306 as illustrated in FIG. 3) may also be configured to execute modules by software, hardware, firmware, or any combinations thereof, and other mechanisms for configuring processing capabilities on the processors. As described in the present disclosure, "module" may refer to any component or set of components that perform the functionality attributed to the module. It may include one or more physical processors during the execution of processor-readable instructions, circuitry, hardware, storage media, and/or any other components.

By way of another example, memory described in the present disclosure (e.g., memory 8 and 13 as illustrated in FIG. 1, and electronic storage 304 as illustrated in FIG. 3) can be a single memory or one or more memories or memory locations that include, but are not limited to, a random access memory ("RAM"), a memory buffer, a hard drive, a database, an erasable programmable read-only memory ("EPROM"), an electrically erasable programmable read-only memory ("EEPROM"), a read-only memory ("ROM"), a flash memory, hard disk or any other various layers of a memory hierarchy. Memory 8 can be used to store any type of instructions and patient data associated with algorithms, processes, or operations for controlling the general functions and operations of the physiological monitoring device.

Additionally, the electronic device (e.g., physiological monitoring device 7 and monitor mount 10 as illustrated in FIG. 1, device 204 as illustrated in FIG. 2, and device 302 as illustrated in FIG. 3) includes a display/GUI. As illustrated in FIG. 1, for example, the display/GUI 4 is for displaying various patient data and hospital or patient care information and includes a user interface implemented for allowing communication between a user and the physiological monitoring device 7. The display/GUI 4 includes, but is not limited to, a keyboard, a liquid crystal display ("LCD"), cathode ray tube ("CRT"), thin-film transistor ("TFT"), light-emitting diode ("LED"), high definition ("HD"), or other similar display devices with touch screen capabilities. The patient information displayed can, for example, relate to the measured physiological parameters of patient 1 (e.g., blood pressure, heart-related information, pulse oximetry, respiration information, etc.) as well as information related to the transporting of the patient 1 (e.g., transport indicators).

a computer-readable medium can comprise DRAM, RAM, ROM, EEPROM, CD-ROM or other optical disk storage, magnetic disk storage or other magnetic storage devices, or any other medium that can be used to carry or store desired computer-readable program code in the form of instructions or data structures and that can be accessed by a general-purpose or special-purpose computer, or a general-purpose or special-purpose processor. Disk or disc, as used herein, include compact disc ("CD"), laser disc, optical disc, digital versatile disc ("DVD"), floppy disk, and Blu-ray disc where disks usually reproduce data magnetically, while discs reproduce data optically with lasers. Combinations of the above are also included within the scope of computer-readable media.

The detailed description is made with reference to the accompanying drawings and is provided to assist in a comprehensive understanding of various example embodiments of the present disclosure. Changes may be made in the function and arrangement of elements discussed without departing from the scope of the disclosure. Various embodiments may omit, substitute, or add various procedures or components as appropriate. For instance, features described with respect to certain embodiments may be combined in other embodiments. In addition, descriptions of well-known functions and constructions may be omitted for clarity and conciseness. Accordingly, those of ordinary skill in the art will recognize that various changes and modifications of the examples described herein can be made without departing from the scope of the present disclosure.

Use of the phrases "capable of," "capable to," "operable to," or "configured to" in one or more embodiments, refers to some apparatus, logic, hardware, and/or element designed in such a way to enable the use of the apparatus, logic, hardware, and/or element in a specified manner. Use of the phrase "exceed" in one or more embodiments, indicates that a measured value could be higher than a pre-determined threshold (e.g., an upper threshold), or lower than a pre-determined threshold (e.g., a lower threshold). When a pre-determined threshold range (defined by an upper threshold and a lower threshold) is used, the use of the phrase "exceed" in one or more embodiments could also indicate a measured value is outside the pre-determined threshold range (e.g., higher than the upper threshold or lower than the lower threshold). The subject matter of the present disclosure is provided as examples of apparatus, systems, methods, circuits, and programs for performing the features described in the present disclosure. However, further features or variations are contemplated in addition to the features described above. It is contemplated that the implementation of the components and functions of the present disclosure can be done with any newly arising technology that may replace any of the above-implemented technologies.

Various modifications to the disclosure will be readily apparent to those skilled in the art, and the generic principles defined herein may be applied to other variations without departing from the scope of the present disclosure. Throughout the present disclosure the terms "example," "examples," or "exemplary" indicate examples or instances and do not imply or require any preference for the noted examples. Thus, the present disclosure is not to be limited to the examples and designs described herein but is to be accorded the widest scope consistent with the principles and novel features disclosed. The invention is defined by the appended claims.

## Claims

1. An apparatus (7) for determining artifact signals from a plurality of sample signals collected during a pre-determined time window from at least one electrocardiogram, "ECG", lead (12) to be affixed to a patient (1), the apparatus comprising:
one or more processors (3); and
a computer-readable medium (8) storing instructions that, when executed by the one or more processors, perform operations comprising:
selecting a plurality of sample points from the plurality of sample signals,
extracting a plurality of features from the selected plurality of sample points,
generating a probability of existence of the artifact signals in the plurality of sample signals, by applying at least two of the plurality of features as inputs to an artificial neural network, "ANN," (404),
identifying a plurality of QRS-complexes from the plurality of sample signals,
extracting one or more features corresponding to the identified plurality of QRS-complexes,
generating a signal quality index, "SQI", by comparing the one or more features corresponding to the identified plurality of QRS-complexes, wherein the one or more extracted features corresponding to the plurality of QRS-complexes comprise:
an amplitude of an R-peak in each of the plurality of QRS-complexes,
a width of the R-peak in each of the plurality of QRS-complexes,
an R-R interval of consecutive R-peaks included in the plurality of QRS-complexes,
an area of each of the plurality of QRS-complexes, or
an area of an S region in each of the plurality of QRS-complexes;
and determining the artifact signals based on the probability, and in the case that the probability determination deemed the sample signals to include artifacts, further determining the artifact signals based on the SQI;
and wherein the determination of the SQI by comparing the one or more features corresponding to the plurality of QRS-complexes further comprises:
clustering the plurality of QRS-complexes into a first cluster and a second cluster, the second cluster having a larger number of QRS-complexes than the first cluster, and
determining the SQI by comparing the one or more extracted features corresponding to each of the plurality of QRS-complexes within the second cluster.

2. The apparatus of claim 1, wherein the selected plurality of sample points comprises at least one of:
a sample point with a maximum amplitude,
a sample point with a minimum amplitude, and
a sample point exceeding a pre-determined threshold.

3. The apparatus of claim 1, wherein the plurality of features extracted from the selected plurality of sample points comprises:
an amplitude difference between a sample point with a maximum amplitude and a sample point with a minimum amplitude,
a medium amplitude of the selected plurality of sample points,
a standard deviation of the selected plurality of sample points,
an amplitude difference between two consecutive sample points, or
a number of two consecutive sample points when the amplitude difference between the two consecutive sample points exceeds a pre-determined threshold.

4. The apparatus of claim 1, wherein the SQI is calculated by comparing the similarities in the one or more extracted features corresponding to the plurality of QRS-complexes.

5. The apparatus of claim 4, wherein, when the SQI is below a pre-determined threshold, the sample signals are determined to have the artifact signals.

6. The apparatus of claim 1, wherein when the SQI is below a pre-determined threshold, the computer-readable medium storing instructions that, when executed by the one or more processors, further performs operations comprising:
determining one or more fiducial points corresponding to the same QRS-complex measured from two or more ECG leads; and
determining the same QRS-complex as a valid QRS-complex when a difference between the one or more fiducial points measured from the two or more ECG leads is below a pre-determined threshold.

7. The apparatus of claim 1, wherein, when the SQI is below a pre-determined threshold, the operations further comprise:
determining an onset point and an offset point of the same QRS-complex measured from two or more ECG leads;
based on the onset point and the offset point, determining an area of the same QRS-complex measured from each of the two or more ECG leads; and
determining the same QRS-complex as a valid QRS-complex when an overlap in the area of the same QRS-complex measured from each of the two or more ECG leads exceeds a pre-determined threshold.

8. A computer-implemented method for determining artifact signals from a plurality of sample signals collected during a pre-determined time window from at least one electrocardiogram, "ECG", lead (12) to be affixed to a patient (1), the method comprising the steps of:
selecting a plurality of sample points from in the plurality of sample signals;
extracting a plurality of features from the selected plurality of sample points;
generating a probability of existence of the artifact signals in the plurality of sample signals, by applying at least two of the plurality of features as inputs to an artificial neural network, "ANN", (404);
identifying a plurality of QRS-complexes from the plurality of sample signals; and
determining the artifact signals based on the probability, and in the case that the probability determination deemed the sample signals to include artifacts, further determining the artifact signals based on a signal quality index, "SQI",;
wherein the SQI is calculated by comparing the similarities in the extracted one or more features corresponding to the plurality of QRS-complexes;
and wherein the step of determining the SQI by comparing the one or more features corresponding to the plurality of QRS-complexes further comprises:
clustering the plurality of QRS-complexes into a first cluster and a second cluster, the second clustering having a larger number of QRS-complexes than the first cluster, and
determining the SQI by comparing the one or more extracted features corresponding to each of the plurality of QRS-complexes within the second cluster;
wherein the one or more extracted features corresponding to the plurality of QRS-complexes comprises:
an amplitude of an R-peak in each of the plurality of QRS-complexes,
a width of the R-peak in each of the plurality of QRS-complexes,
an R-R interval of consecutive R-peaks included in the plurality of QRS-complexes,
an area of each of the plurality of QRS-complexes, or
an area of an S region in each of the plurality of QRS-complexes.

9. The method of claim 8, wherein the selected plurality of sample points comprises:
a sample point with a maximum amplitude,
a sample point with a minimum amplitude, or
a sample point exceeding a pre-determined threshold value.

10. The method of claim 8, wherein the plurality of features extracted from the selected plurality of sample points comprises:
an amplitude difference between a sample point with a maximum amplitude and a sample point with a minimum amplitude,
a medium amplitude value of the selected plurality of sample points,
a standard deviation of the selected plurality of sample points,
an amplitude difference between two consecutive sample points, or
a number of the two consecutive sample points when the amplitude difference between the two consecutive sample points exceeds a pre-determined threshold.

11. The method of claim 8, wherein when the SQI is below a pre-determined threshold, the sample signals are determined to have the artifact signals.

12. The method of claim 8, wherein when the SQI is below a pre-determined threshold, the method further comprising the steps of:
determining one or more fiducial points corresponding to the same QRS-complex measured from two or more ECG leads; and
determining the same QRS-complex as a valid QRS-complex when a difference between the one or more fiducial points measured from the two or more ECG leads is below a pre-determined threshold.

13. The method of claim 8, wherein when the SQI is below a pre-determined threshold, the method further comprising:
determining an onset point and an offset point of the same QRS-complex measured from two or more ECG leads;
based on the onset point and the offset point, determining an area of the same QRS-complex measured from each of the two or more ECG leads; and
determining the same QRS-complex as a valid QRS-complex when an overlap in the area of the same QRS-complex measured from each of the two or more ECG leads exceeds a pre-determined threshold.

14. The method of claim 13, further comprising the steps of:
determining a plurality of heart rate values based on R-R intervals measured from the plurality of QRS-complexes;
ranking the plurality of heart rate values;
comparing a new heart rate calculated based on the valid QRS-complex with a medium heart rate of the ranked plurality of heart rate values; and
calculating an output heart rate based on the new heart rate and the medium heart rate value.

15. The method of claim 13, further comprising the steps of:
extracting one or more features of the valid QRS-complex, the one or more features including an R-R interval, an amplitude of an R-wave, a width of the R-wave, an amplitude of a P-wave, a polarity of an ST-segment;
establishing an adjusted threshold corresponding to the one or more features of the valid QRS-complex; and
when the one or more features of the valid QRS-complex exceed the adjusted threshold, storing the valid QRS-complex into a QRS-complex template database.

## Patentansprüche

1. Vorrichtung (7) zum Bestimmen von Artefaktsignalen aus einer Mehrzahl von Abtastsignalen, die während eines vorbestimmten Zeitfensters von mindestens einer Elektrokardiogramm, "EKG", -Elektrode (12), die am Patienten (1) anzubringen ist, erfasst werden, wobei die Vorrichtung umfasst:
einen oder mehrere Prozessoren (3); und
ein computerlesbares Medium (8), das Anweisungen speichert, die, wenn sie durch die ein oder mehreren Prozessoren ausgeführt werden, Operationen durchführen, die umfassen:
Auswählen einer Mehrzahl von Abtastpunkten aus der Mehrzahl von Abtastsignalen,
Extrahieren einer Mehrzahl von Merkmalen aus der ausgewählten Mehrzahl von Abtastpunkten,
Erzeugen einer Wahrscheinlichkeit für das Vorhandensein der Artefaktsignale in der Mehrzahl von Abtastsignalen, indem mindestens zwei der Mehrzahl von Merkmalen als Eingaben in ein künstliches neuronales Netz, "ANN" (Artificial Neural Network), (404), verwendet werden, Identifizieren einer Mehrzahl von QRS-Komplexen aus der Mehrzahl von Abtastsignalen,
Extrahieren eines oder mehrerer Merkmale, die der identifizierten Mehrzahl von QRS-Komplexen entsprechen, Erzeugen eines Signalqualitätsindex, "SQI", durch Vergleichen der ein oder mehreren Merkmale, die der identifizierten Mehrzahl von QRS-Komplexen entsprechen, wobei die ein oder mehreren extrahierten Merkmale, die der Mehrzahl von QRS-Komplexen entsprechen, umfassen:
eine Amplitude einer R-Zacke in jedem der Mehrzahl von QRS-Komplexen,
eine Breite der R-Zacke in jedem der Mehrzahl von QRS-Komplexen,
ein R-R-Intervall von aufeinanderfolgenden R-Zacken, die in der Mehrzahl von QRS-Komplexen enthalten sind,
eine Fläche jedes der Mehrzahl von QRS-Komplexen, oder
eine Fläche einer S-Region in jedem der Mehrzahl von QRS-Komplexen;
und Bestimmen der Artefaktsignale basierend auf der Wahrscheinlichkeit, und in dem Fall, dass die Wahrscheinlichkeitsbestimmung ergab, dass die Abtastsignale Artefakte enthalten, ferner Bestimmen der Artefaktsignale basierend auf dem SQI;
und wobei das Bestimmen des SQI durch Vergleichen der ein oder mehreren Merkmale, die der Mehrzahl von QRS-Komplexen entsprechen, ferner umfasst:
Zusammenfassen der Mehrzahl von QRS-Komplexen zu einem ersten Cluster und einem zweiten Cluster, wobei der zweite Cluster eine größere Anzahl von QRS-Komplexen aufweist als der erste Cluster, und
Bestimmen des SQI durch Vergleichen der ein oder mehreren extrahierten Merkmale, die jedem der Mehrzahl von QRS-Komplexen im zweiten Cluster entsprechen.

2. Vorrichtung nach Anspruch 1, wobei die ausgewählte Mehrzahl von Abtastpunkten mindestens eines der folgenden umfasst:
einen Abtastpunkt mit einer maximalen Amplitude,
einen Abtastpunkt mit einer minimalen Amplitude, und
einen Abtastpunkt, der einen vorbestimmten Schwellenwert übersteigt.

3. Vorrichtung nach Anspruch 1, wobei die Mehrzahl von Merkmalen, die aus der ausgewählten Mehrzahl von Abtastpunkten extrahiert werden, umfasst:
eine Amplitudendifferenz zwischen einem Abtastpunkt mit maximaler Amplitude und einem Abtastpunkt mit minimaler Amplitude,
eine mittlere Amplitude der ausgewählten Mehrzahl von Abtastpunkten,
eine Standardabweichung der ausgewählten Mehrzahl von Abtastpunkten,
eine Amplitudendifferenz zwischen zwei aufeinanderfolgenden Abtastpunkten, oder
eine Anzahl von zwei aufeinanderfolgenden Abtastpunkten, wenn die Amplitudendifferenz zwischen den beiden aufeinanderfolgenden Abtastpunkten einen vorbestimmten Schwellenwert übersteigt.

4. Vorrichtung nach Anspruch 1, wobei der SQI durch Vergleichen der Ähnlichkeiten in den ein oder mehreren extrahierten Merkmalen, die der Mehrzahl von QRS-Komplexen entsprechen, berechnet wird.

5. Vorrichtung nach Anspruch 4, wobei, wenn der SQI unter einem vorbestimmten Schwellenwert liegt, bestimmt wird, dass die Abtastsignale Artefaktsignale aufweisen.

6. Vorrichtung nach Anspruch 1, wobei, wenn der SQI unter einem vorbestimmten Schwellenwert liegt, das computerlesbare Medium Anweisungen speichert, die, wenn sie durch die ein oder mehreren Prozessoren ausgeführt werden, ferner Operationen durchführen, die umfassen:
Bestimmen eines oder mehrerer Referenzpunkte, die demselben QRS-Komplex entsprechen, der von zwei oder mehr EKG-Elektroden gemessen wird; und
Bestimmen desselben QRS-Komplexes als gültigen QRS-Komplex, wenn eine Differenz zwischen den ein oder mehreren Referenzpunkten, die von den zwei oder mehreren EKG-Elektroden gemessen werden, unter einem vorbestimmten Schwellenwert liegt.

7. Vorrichtung nach Anspruch 1, wobei, wenn der SQI unter einem vorbestimmten Schwellenwert liegt, die Operationen ferner umfassen:
Bestimmen eines Onset (Anfangs) -Punktes und eines Offset (End) -Punktes desselben QRS-Komplexes, der von zwei oder mehr EKG-Elektroden gemessen wird;
basierend auf dem Onset-Punkt und dem Offset-Punkt, Bestimmen einer Fläche desselben QRS-Komplexes, der durch jede der zwei oder mehr EKG-Elektroden gemessen wird; und
Bestimmen desselben QRS-Komplexes als gültigen QRS-Komplex, wenn eine Überlappung in der Fläche desselben QRS-Komplexes, der durch jede der zwei oder mehr EKG-Elektroden gemessen wird, einen vorbestimmten Schwellenwert übersteigt.

8. Computer-implementiertes Verfahren zum Bestimmen von Artefaktsignalen aus einer Mehrzahl von Abtastsignalen, die während eines vorbestimmten Zeitfensters von mindestens einer Elektrokardiogramm, "EKG", -Elektrode (12), die am Patienten (1) anzubringen ist, erfasst werden, wobei das Verfahren die Schritte umfasst:
Auswählen einer Mehrzahl von Abtastpunkten in der Mehrzahl von Abtastsignalen;
Extrahieren einer Mehrzahl von Merkmalen aus der ausgewählten Mehrzahl von Abtastpunkten;
Erzeugen einer Wahrscheinlichkeit für das Vorhandensein der Artefaktsignale in der Mehrzahl von Abtastsignalen, indem mindestens zwei der Mehrzahl von Merkmalen als Eingaben in ein künstliches neuronales Netz, "ANN", (404), verwendet werden;
Identifizieren einer Mehrzahl von QRS-Komplexen aus der Mehrzahl von Abtastsignalen; und
Bestimmen der Artefaktsignale basierend auf der Wahrscheinlichkeit, und in dem Fall, dass die Wahrscheinlichkeitsbestimmung ergab, dass die Abtastsignale Artefakte enthalten, ferner Bestimmen der Artefaktsignale basierend auf einem Signalqualitätsindex, "SQI";
wobei der SQI durch Vergleichen der Ähnlichkeiten in den extrahierten ein oder mehreren Merkmalen, die der Mehrzahl von QRS-Komplexen entsprechen, berechnet wird;
und wobei der Schritt des Bestimmens des SQI durch Vergleichen der ein oder mehreren Merkmale, die der Mehrzahl von QRS-Komplexen entsprechen, ferner umfasst:
Zusammenfassen der Mehrzahl von QRS-Komplexen zu einem ersten Cluster und einem zweiten Cluster, wobei der zweite Cluster eine größere Anzahl von QRS-Komplexen aufweist als der erste Cluster, und
Bestimmen des SQI durch Vergleichen der ein oder mehreren extrahierten Merkmale, die jedem der Mehrzahl von QRS-Komplexen im zweiten Cluster entsprechen;
wobei die ein oder mehreren extrahierten Merkmale, die der Mehrzahl von QRS-Komplexen entsprechen, umfassen:
eine Amplitude einer R-Zacke in jedem der Mehrzahl von QRS-Komplexen,
eine Breite der R-Zacke in jedem der Mehrzahl von QRS-Komplexen,
ein R-R-Intervall von aufeinanderfolgenden R-Zacken, die in der Mehrzahl von QRS-Komplexen enthalten sind,
eine Fläche jedes der Mehrzahl von QRS-Komplexen, oder
eine Fläche einer S-Region in jedem der Mehrzahl von QRS-Komplexen.

9. Verfahren nach Anspruch 8, wobei die ausgewählte Mehrzahl von Abtastpunkten umfasst:
einen Abtastpunkt mit einer maximalen Amplitude,
einen Abtastpunkt mit einer minimalen Amplitude, oder
einen Abtastpunkt, der einen vorbestimmten Schwellenwert übersteigt.

10. Verfahren nach Anspruch 8, wobei die Mehrzahl von Merkmalen, die aus der ausgewählten Mehrzahl von Abtastpunkten extrahiert werden, umfasst:
eine Amplitudendifferenz zwischen einem Abtastpunkt mit maximaler Amplitude und einem Abtastpunkt mit minimaler Amplitude,
einen mittleren Amplitudenwert der ausgewählten Mehrzahl von Abtastpunkten,
eine Standardabweichung der ausgewählten Mehrzahl von Abtastpunkten,
eine Amplitudendifferenz zwischen zwei aufeinanderfolgenden Abtastpunkten, oder
eine Anzahl der zwei aufeinanderfolgenden Abtastpunkte, wenn die Amplitudendifferenz zwischen den beiden aufeinanderfolgenden Abtastpunkten einen vorbestimmten Schwellenwert übersteigt.

11. Verfahren nach Anspruch 8, wobei, wenn der SQI unter einem vorbestimmten Schwellenwert liegt, bestimmt wird, dass die Abtastsignale Artefaktsignale aufweisen.

12. Verfahren nach Anspruch 8, wobei, wenn der SQI unter einem vorbestimmten Schwellenwert liegt, das Verfahren ferner die Schritte umfasst:
Bestimmen eines oder mehrerer Referenzpunkte, die demselben QRS-Komplex entsprechen, der von zwei oder mehr EKG-Elektroden gemessen wird; und
Bestimmen desselben QRS-Komplexes als gültigen QRS-Komplex, wenn eine Differenz zwischen den ein oder mehreren Referenzpunkten, die von den zwei oder mehreren EKG-Elektroden gemessen werden, unter einem vorbestimmten Schwellenwert liegt.

13. Verfahren nach Anspruch 8, wobei, wenn der SQI unter einem vorbestimmten Schwellenwert liegt, das Verfahren ferner umfasst:
Bestimmen eines Onset (Anfangs) -Punktes und eines Offset (End) -Punktes desselben QRS-Komplexes, der von zwei oder mehr EKG-Elektroden gemessen wird;
basierend auf dem Onset-Punkt und dem Offset-Punkt, Bestimmen einer Fläche desselben QRS-Komplexes, der durch jede der zwei oder mehr EKG-Elektroden gemessen wird; und
Bestimmen desselben QRS-Komplexes als gültigen QRS-Komplex, wenn eine Überlappung in der Fläche desselben QRS-Komplexes, der durch jede der zwei oder mehr EKG-Elektroden gemessen wird, einen vorbestimmten Schwellenwert übersteigt.

14. Verfahren nach Anspruch 13, ferner die Schritte umfassend:
Bestimmen einer Mehrzahl von Herzfrequenzwerten basierend auf den gemessenen R-R-Intervallen aus der Mehrzahl von QRS-Komplexen;
Einstufen der Mehrzahl von Herzfrequenzwerten;
Vergleichen einer neuen Herzfrequenz, die basierend auf dem gültigen QRS-Komplex berechnet wird, mit einer mittleren Herzfrequenz aus der eingestuften Mehrzahl von Herzfrequenzwerten; und
Berechnen einer Ausgangsherzfrequenz basierend auf der neuen Herzfrequenz und dem mittleren Herzfrequenzwert.

15. Verfahren nach Anspruch 13, ferner die Schritte umfassend:
Extrahieren eines oder mehrerer Merkmale des gültigen QRS-Komplexes, wobei die ein oder mehreren Merkmale ein R-R-Intervall, eine Amplitude einer R-Welle, eine Breite der R-Welle, eine Amplitude einer P-Welle und eine Polarität eines ST-Segments beinhalten;
Festlegen eines angepassten Schwellenwerts, der den ein oder mehreren Merkmalen des gültigen QRS-Komplexes entspricht; und
wenn die ein oder mehreren Merkmale des gültigen QRS-Komplexes den angepassten Schwellenwert überschreiten, Speichern des gültigen QRS-Komplexes in einer QRS-Komplex-Vorlagendatenbank.

## Revendications

1. Appareil (7) pour déterminer des signaux d'artefacts à partir d'une pluralité de signaux d'échantillon recueillis pendant une fenêtre temporelle prédéterminée à partir d'au moins une dérivation d'électrocardiogramme, « ECG » (12) à fixer sur un patient (1), l'appareil comprenant :
un ou plusieurs processeurs (3) ; et
un support lisible par ordinateur (8) stockant des instructions qui, lorsqu'elles sont exécutées par le ou les processeurs, effectuent des opérations comprenant :
la sélection d'une pluralité de points d'échantillon à partir de la pluralité de signaux d'échantillon,
l'extraction d'une pluralité de caractéristiques à partir de la pluralité de points d'échantillon sélectionnés,
la génération d'une probabilité d'existence des signaux d'artefact dans la pluralité de signaux d'échantillon, en appliquant au moins deux de la pluralité de caractéristiques comme entrées à un réseau neuronal artificiel, « ANN » (404),
l'identification d'une pluralité de complexes QRS à partir de la pluralité de signaux d'échantillon,
l'extraction d'une ou plusieurs caractéristiques correspondant à la pluralité de complexes QRS identifiés, la génération d'un indice de qualité de signal, « SQI », en comparant la ou les caractéristiques correspondant à la pluralité identifiée de complexes QRS, la ou les caractéristiques extraites correspondant à la pluralité de complexes QRS comprenant :
une amplitude d'un pic R dans chacun de la pluralité de complexes QRS,
une largeur du pic R dans chacun de la pluralité de complexes QRS,
un intervalle R-R de pics R consécutifs inclus dans la pluralité de complexes QRS,
une zone de chacun de la pluralité de complexes QRS, ou une zone d'une région S dans chacun de la pluralité de complexes QRS ;
et la détermination des signaux d'artefacts sur la base de la probabilité, et dans le cas où la détermination de la probabilité a jugé que les signaux de l'échantillon comprenaient des artefacts, la détermination en outre des signaux d'artefacts sur la base du SQI ;
et la détermination du SQI par comparaison de la ou des caractéristiques correspondant à la pluralité de complexes QRS comprenant en outre :
le regroupement de la pluralité de complexes QRS en une première grappe et une seconde grappe, la seconde grappe ayant un plus grand nombre de complexes QRS que la première grappe, et
la détermination du SQI en comparant la ou les caractéristiques extraites correspondant à chacun de la pluralité de complexes QRS dans la seconde grappe.

2. Appareil selon la revendication 1, la pluralité de points d'échantillon sélectionnés comprenant au moins un point d'échantillon parmi :
un point d'échantillon avec une amplitude maximale,
un point d'échantillon avec une amplitude minimale, et
un point d'échantillon dépassant un seuil prédéterminé.

3. Appareil selon la revendication 1, la pluralité de caractéristiques extraites à partir de la pluralité de points d'échantillon sélectionnés comprenant :
une différence d'amplitude entre un point d'échantillon ayant une amplitude maximale et un point d'échantillon ayant une amplitude minimale,
une amplitude moyenne de la pluralité de points d'échantillon sélectionnés,
un écart-type de la pluralité de points d'échantillon sélectionnés,
une différence d'amplitude entre deux points d'échantillon consécutifs, ou
un nombre des deux points d'échantillon consécutifs lorsque la différence d'amplitude entre les deux points d'échantillon consécutifs dépasse un seuil prédéterminé.

4. Appareil selon la revendication 1, le SQI étant calculé en comparant les similitudes dans la ou les caractéristiques extraites correspondant à la pluralité de complexes QRS.

5. Appareil selon la revendication 4, lorsque le SQI est inférieur à un seuil prédéterminé, les signaux d'échantillon étant déterminés comme ayant les signaux d'artefact.

6. Appareil selon la revendication 1, lorsque le SQI est inférieur à un seuil prédéterminé, le support lisible par ordinateur stockant des instructions qui, lorsqu'elles sont exécutées par le ou les processeurs, effectuent en outre des opérations comprenant :
la détermination d'un ou plusieurs points de repère correspondant au même complexe QRS mesuré à partir de deux ou plus de deux dérivations ECG ; et
la détermination du même complexe QRS comme un complexe QRS valide lorsqu'une différence entre le ou les points de repère mesurés à partir des deux ou plus de deux dérivations ECG est inférieure à un seuil prédéterminé.

7. Appareil selon la revendication 1, lorsque le SQI est inférieur à un seuil prédéterminé, les opérations comprenant en outre :
la détermination d'un point de départ et d'un point de décalage du même complexe QRS mesuré à partir de deux ou plus de deux dérivations ECG ;
sur la base du point de départ et du point de décalage, la détermination d'une zone du même complexe QRS mesurée à partir de chacune des deux ou plus de deux dérivations ECG ; et
la détermination du même complexe QRS comme un complexe QRS valide lorsqu'un chevauchement de la zone du même complexe QRS mesuré à partir de chacune des deux ou plus de deux dérivations ECG dépasse un seuil prédéterminé.

8. Procédé mis en œuvre par ordinateur pour déterminer des signaux d'artefact à partir d'une pluralité de signaux d'échantillon recueillis pendant une fenêtre de temps prédéterminée à partir d'au moins une dérivation d'électrocardiogramme, « ECG » (12) à fixer sur un patient (1), le procédé comprenant les étapes de :
sélection d'une pluralité de points d'échantillon à partir de la pluralité de signaux d'échantillon ;
extraction d'une pluralité de caractéristiques à partir de la pluralité de points d'échantillon sélectionnés ;
génération d'une probabilité d'existence des signaux d'artefact dans la pluralité de signaux d'échantillon, en appliquant au moins deux de la pluralité de caractéristiques comme entrées à un réseau neuronal artificiel, « ANN » (404),
identification d'une pluralité de complexes QRS à partir de la pluralité de signaux d'échantillon ; et
détermination des signaux d'artefacts sur la base de la probabilité, et dans le cas où la détermination de la probabilité a jugé que les signaux d'échantillon comprenaient des artefacts, détermination en outre des signaux d'artefacts sur la base d'un indice de qualité de signal, « SQI » ;
le SQI étant calculé en comparant les similitudes dans la ou les caractéristiques extraites correspondant à la pluralité de complexes QRS ;
et l'étape comprenant la détermination du SQI en comparant la ou les caractéristiques correspondant à la pluralité de complexes QRS comprenant en outre :
le regroupement de la pluralité de complexes QRS en une première grappe et une seconde grappe, la seconde grappe ayant un plus grand nombre de complexes QRS que la première grappe, et
la détermination du SQI en comparant la ou les caractéristiques extraites correspondant à chacun de la pluralité de complexes QRS dans la seconde grappe ;
la ou les caractéristiques extraites correspondant à la pluralité de complexes QRS comprenant :
une amplitude d'un pic R dans chacun de la pluralité de complexes QRS,
une largeur du pic R dans chacun de la pluralité de complexes QRS,
un intervalle R-R de pics R consécutifs inclus dans la pluralité de complexes QRS,
une zone de chacun de la pluralité de complexes QRS, ou une zone d'une région S dans chacun de la pluralité de complexes QRS.

9. Procédé selon la revendication 8, la pluralité de points d'échantillon sélectionnés comprenant :
un point d'échantillon avec une amplitude maximale,
un point d'échantillon avec une amplitude minimale, ou
un point d'échantillon dépassant une valeur seuil prédéterminée.

10. Procédé selon la revendication 8, la pluralité de caractéristiques extraites à partir de la pluralité de points d'échantillon sélectionnés comprenant :
une différence d'amplitude entre un point d'échantillon ayant une amplitude maximale et un point d'échantillon ayant une amplitude minimale,
une valeur d'amplitude moyenne de la pluralité de points d'échantillon sélectionnés,
un écart-type de la pluralité de points d'échantillon sélectionnés,
une différence d'amplitude entre deux points d'échantillon consécutifs, ou
un nombre des deux points d'échantillon consécutifs lorsque la différence d'amplitude entre les deux points d'échantillon consécutifs dépasse un seuil prédéterminé.

11. Procédé selon la revendication 8, lorsque le SQI est inférieur à un seuil prédéterminé, les signaux d'échantillon étant déterminés comme ayant les signaux d'artefact.

12. Procédé selon la revendication 8, lorsque le SQI est inférieur à un seuil prédéterminé, le procédé comprenant en outre les étapes de :
détermination d'un ou plusieurs points de repère correspondant au même complexe QRS mesuré à partir de deux ou plus de deux dérivations ECG ; et
détermination du même complexe QRS comme un complexe QRS valide lorsqu'une différence entre le ou les points de repère mesurés à partir des deux ou plus de deux dérivations ECG est inférieure à un seuil prédéterminé.

13. Procédé selon la revendication 8, lorsque le SQI est inférieur à un seuil prédéterminé, le procédé comprenant en outre :
la détermination d'un point de départ et d'un point de décalage du même complexe QRS mesuré à partir de deux ou plus de deux dérivations ECG ;
sur la base du point de départ et du point de décalage, la détermination d'une zone du même complexe QRS mesurée à partir de chacune des deux ou plus de deux dérivations ECG ; et
la détermination du même complexe QRS comme un complexe QRS valide lorsqu'un chevauchement de la zone du même complexe QRS mesuré à partir de chacune des deux ou plus de deux dérivations ECG dépasse un seuil prédéterminé.

14. Procédé selon la revendication 13, comprenant en outre les étapes de :
détermination d'une pluralité de valeurs de fréquence cardiaque sur la base des intervalles R-R mesurés à partir de la pluralité de complexes QRS ;
classement de la pluralité de valeurs de fréquence cardiaque ;
comparaison d'une nouvelle fréquence cardiaque calculée sur la base du complexe QRS valide avec une fréquence cardiaque moyenne de la pluralité de valeurs de fréquence cardiaque classées ; et
calcul d'une fréquence cardiaque de sortie sur la base de la nouvelle fréquence cardiaque et de la valeur de fréquence cardiaque moyenne.

15. Procédé selon la revendication 13, comprenant en outre les étapes de :
extraction d'une ou plusieurs caractéristiques du complexe QRS valide, la ou les caractéristiques comprenant un intervalle R-R, une amplitude d'une onde R, une largeur de l'onde R, une amplitude d'une onde P, une polarité d'un segment ST ;
établissement d'un seuil ajusté correspondant à la ou aux caractéristiques du complexe QRS valide ; et
lorsque la ou les caractéristiques du complexe QRS valide dépassent le seuil ajusté, stockage du complexe QRS valide dans une base de données de modèles de complexes QRS.
